# EUROPEAN PATENT APPLICATION

(11) **EP 1 217 642 A1**
(43) Date of publication of application: **26.06.2002**
(21) Application number: 00850220.5
(22) Date of filing: 22.12.2000
(51) Int. Cl.: H01J 35/32, A61N 5/10

(54) **Active cooling of a miniature x-ray tube**

(71) Applicant: Radi Medical Technologies AB, 754 50 Uppsala (SE)
(72) Inventor: Tirén, Jonas, 753 34 Uppsala (SE)
(74) Representative: Holmberg, Nils Anders Patrik

(57) **Abstract**

Elongated x-ray tube unit comprising a miniaturized x-ray tube (2) arranged in a distal end of the unit adapted to generate x-ray radiation at a treatment position within a patient's body. The x-ray tube unit further comprises a closed cooling solution supplying system provided with tubing means (24) arranged within the x-ray tube unit and adapted to provide a cooling solution to the vicinity of the x-ray tube in order to cool the x-ray tube.

## Description

### FIELD OF THE INVENTION

The present invention relates to an elongated x-ray tube unit comprising a miniature x-ray tube according to the preamble of the independent claim.

A miniature x-ray tube according to the invention is, for example, useful in applications for prevention of restenosis and for treating diseases, such as cancer, in a living body.

### BACKGROUND OF THE INVENTION

In treating stenosis in coronary arteries, a restenosis occurs in 30-60% of the cases. It is known that a treatment with beta- or gamma- (x-ray) radiation will decrease the occurrence of restenosis substantially.

Another example of an application of the present invention is treatment of cancer tumors where it is desired to deliver radiation locally.

Methods to apply the x-ray radiation to the site of treatment are presently subject to intensive research. For example, the use of radioactive pellets or balloons etc. to introduce radioactive isotopes is known. Since this method has some drawbacks, such as the need for substantial efforts to control radiation in the environment outside the patient and problems with dose control, the use of a miniature electrical x-ray tube including a cold cathode has been proposed. Such a tube may be switched on and off due to its electrical activation. An example of such a x-ray tube is described in the US patent 5,854,822.

However, the conventional miniature electrical x-ray tubes exhibit a problem in that the x-ray tube generates heat. In order not to damage the surrounding tissue, the temperature of the x-ray tube at the interface with the tissue should not exceed approximately 40-41 °C.

One way to reduce the generated heat is the use of a pulsed source wherein the electrically activated tube is turned on intermittently. However, a pulsed source exhibit the drawback that the treatment time will be prolonged correspondingly, since the received dose must be held constant. This is costly and increases the discomfort for the patient.

Another way to reduce the generated heat is to apply a sufficiently low current to the conventional x-ray tube. Again, the treatment time has to be correspondingly increased in order to apply the appropriate dose of x-ray radiation, with the same negative consequences as above

Therefore, in order to maintain a short treatment time, cooling the x-ray tube by flushing a saline solution onto the tube is known. Where the x-ray emitter according to the above-mentioned US-5,854,822 is used in a blood vessel, the blood flow past the heat emitter is considered sufficient to dissipate the generated heat. However, if some kind of other cooling mechanism was employed, the power could be increased and the treatment time reduced. Where the device is used in the esophagus or other cavity, a fluid could circulate through a balloon surrounding the heat emitter to prevent damage to the body.

However, with conventional designs cooling by flushing exhibits the problem of enlarging the geometry of the device to be introduced into the body, since the saline must be delivered to the source of heat and therefore must be directed by some means that will occupy space. Also, a flow path through a catheter has to be established and maintained to cool the x-ray tube that is generally awkward. The flow path, typically a tube, will have to be guided along the electrical leads used to power the x-ray tube.

Methods to apply the radiation to the site of treatment are presently subject to intensive research. Generally, a hollow catheter is inserted into the body, typically via an artery, in such a way that its distal end is placed near the site of treatment. A source of radiation attached to the distal end of an elongated member is inserted into the hollow catheter, and is forwarded until the radiation source is disposed at a proper position for radiating the site of treatment. In the specific case of treating cardiac vessels, the catheter is placed near the cardiac vessel tree (this catheter often called a "guide catheter"). A very thin wire - called guide wire - is then used to probe further and reach the site where treatment shall be performed. The therapeutic device is moved along this wire, i.e. by threading the device onto the guide wire. It obvious that the therapeutic device has to have a hole close to its distal end in order to do this.

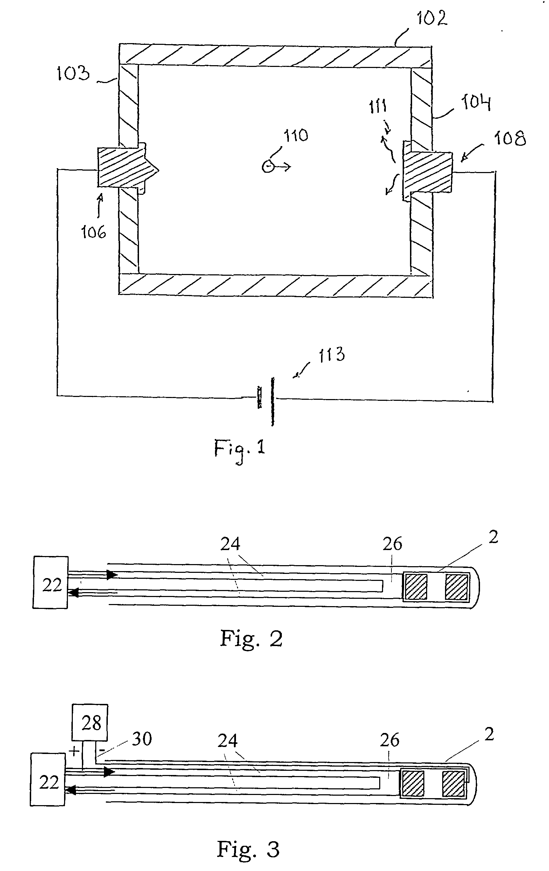

An example of a conventional, prior art miniature x-ray tube is illustrated in the schematic cross-sectional view of figure 1. The tube has an enclosure consisting of a hollow cylindrical tube 102 of a x-ray transparent material (such as glass, silicon carbide, Al₂O₃, quartz, diamond, boron nitride, pyrolytic boron nitride etc.), and end walls 103, 104 (although other arrangements, such as end walls integrated with the tubular enclosure are also known). Although any suitable shape of the enclosure could be used, such as bulb like, spherical or hollow with quadrangular cross section, it is preferred to form the enclosure as a tube, i.e. as a hollow cylinder with a circular cross section or with a similar cross sectional shape, such as hexagonal.
The parts are joined by vacuum tight sealing. This may be achieved by using vacuum grade epoxies, using vacuum brazing with appropriate alloys (typically an Ag/Cu alloy) or by using glass fit. The final assembly must obviously be carried out in a vacuum.

The end walls, which typically are made of a similar material as the enclosure, could in fact be integrated with the tubular enclosure, or even omitted in a case of a tube having an inner diameter of the same order as the diameter of the electrodes.

The enclosure is hermetically sealed, and a vacuum is provided inside the tube. A cathode 106, adapted to emit electrons, and an anode 108, the latter adapted to emit x-ray radiation, penetrates through respectively opposite ends of the tube. When the cathode is connected to the negative potential of a high voltage source 113 and the anode is connected to the corresponding positive potential of the voltage source, electrons 110 will be emitted from the cathode to impact into the anode. As the electrons hit the anode, x-ray radiation 111 is emitted from the anode. The x-ray transparent enclosure allows the x-ray radiation to be delivered to a patient.

The outer diameter of the x-ray tube should generally be within a range of about 0,5 to 3 mm, in order to fit into the vessels for treatment which typically have inner diameters within a range of about 1 to 5 mm. The longitudinal length of a stenosis is typically in the range of 1 to 100 mm.

The treatment duration is selected to provide an adequate radiation dose. For example, a radiation dose suitable for treatment of coronary stenosis typically is in the range of 10-40 Gy, while a radiation dose suitable for treatment of cancer typically is in the range of 10-200 Gy.

One object of the present invention is to achieve an x-ray tube unit provided with a cooling arrangement allowing optimal treatment for the patient with regard to treatment time and radiation dosage.

Another object of the present invention is to achieve an x-ray tube unit avoiding to enlarge the geometry of the device to be introduced into the body.

A further object of the present invention to provide an x-ray tube unit with a miniature electrical x-ray tube having means for providing a cooling solution and electrical power to the x-ray tube.

### SUMMARY OF THE INVENTION

The above-mentioned objects are achieved by an x-ray tube unit according to the characterizing part of the independent claim.

Preferred embodiments are set forth in the dependent claims.

The present invention allows the x-ray tube to operate inside a living body without unduly heating the tissue surrounding the x-ray tube.

With the x-ray tube of the invention, an effective cooling is achieved by circulating a cooling solution in the vicinity of the heat generating part of the x-ray tube.

According to a preferred embodiment of the present invention, the cooling solution is an electrically conductive fluid wherein the x-ray tube is powered via the cooling solution, thereby omitting the need for a separate electrical lead to one of the electrodes. According to this embodiment no saline cooling solution must enter the body since the solution is electrically connected to the therapeutic device.

A further advantage is that no consideration must be taken to how much saline can be allowed to be delivered to the body per time unit.

### BRIEF DESCRIPTION OF DRAWINGS

Figure 1 is a schematic cross-sectional view of an x-ray tube according to the prior art.
Figure 2 is a schematic cross-sectional view of a first embodiment of an x-ray tube unit according to the present invention.
Figure 3 is a schematic cross-sectional view of a second embodiment of an x-ray tube unit according to the present invention.
Figure 4 is a cross-sectional view of the electrical connections to the proximal side including a pumping arrangement according to the second embodiment of the invention.
Figure 5 is a cross-sectional view of the distal end of the tube unit according to the second embodiment of the present invention.

### DETAILED DESCRIPTION OF EMBODIMENTS

The invention shall now be described in detail by way of embodiments. Of course, the described embodiments should not be viewed as limiting for the scope of the invention that is defined by the appended claims.

Figure 2 shows a simplified illustration of an elongated x-ray tube unit according to the present invention. The tube unit comprises an x-ray tube 2 arranged at a distal end of the x-ray tube unit and a cooling solution supplying system 20 that in turn comprises a pumping arrangement 22, tubing means 24 and a cooling location 26 close to the x-ray tube. In figure 2 the cooling location is illustrated as a cooling chamber but may equally be arranged as a cooling conduit.

In order to illustrate only the features directly involved in the preferred embodiment of the present invention the electrical connections to the x-ray tube is not shown in figure 2.

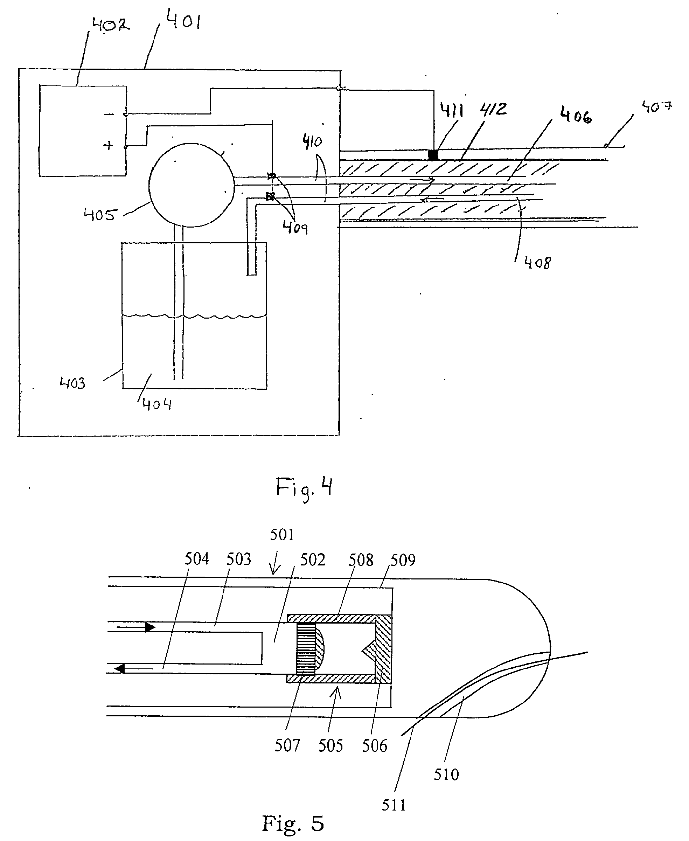

One way to guide the elongated x-ray tube unit to the treatment position is to arrange a so-called guiding extension at the distal end of the tube unit. The guiding extension is provided with a hole (see figure 5) through which a previously inserted guide wire is adapted to run and thereby guide the tube unit to the intended position. Many different types of guiding extensions are described in the prior art that may be applied on a catheter according to the present invention. Since the present invention may be used with any type of guiding extension or even without an extension and because the type of extension used is not directly related to the present invention it is not thoroughly described herein.

The pumping arrangement is arranged at the proximal end of the x-ray tube unit and connected to the tubing means 24 running along the x-ray tube unit to the cooling location 26 close to the x-ray tube, wherein the pumping arrangement supplies the system with cooling solution.
The cooling solution supplying system is closed in the sense that no cooling solution is expelled from the system inside the patient's body.

Figure 3 illustrates the principles of a second embodiment of the present invention where the electrical connection between a power source 28 connected to the proximal end of the tube unit and one of the first and second electrodes of the x-ray tube is achieved via the cooling solution.

A preferably used cooling solution is saline. Since this solution contains ions it is electrically conducting, and can therefore be used as the electrical conductor to one of the poles of the x-ray tube, preferably the proximal pole. Naturally other solutions are possible provided that they are electrically conductive and accepted for use in medical devices inserted into the body. Since the heat is essentially generated by the anode the proximal pole of the tube is preferably the anode. Therefore the anode (the most positive electric potential) is connected to the power supply through the cooling solution, provided essentially through a center lumen in a catheter. The cathode (connected to the negative pole) may be connected by an ordinary conductor, schematically illustrated be reference sign 30, in the form of an insulated wire or by a metal layer, which may be in the form of a braided shield or evaporated or established by a number of known methods known to a person skilled in the technique. One preferred configuration has a coaxial geometry. Finally an outer layer of a biocompatible electrically insulating material is used to insulate the shield from the patient during treatment.

Figure 4 shows an example of how a practical electrical connection is made to the cooling solution. A power unit 401 comprises, among other details, a power source 402, a container 403 for electrically conductive cooling solution 404 and a pump 405. The outlet of the pump is connected to one lumen 406 in the catheter 407. The other lumen 408 is leading the fluid back to the container. Both lumens 406 and 408 are electrically connected to the positive pole of the power source 402 inside the power unit via electrodes 409 in the tubes 410. The reason to connect both lumens is to avoid any electrical potential between the two. It is important to make the electrical connection to the fluid after the pump, especially if the pump is peristaltic, since such a pump may not have continuous flow (in fact the fluid may be interrupted mechanically) and the electrical path will be interrupted consequently. It is naturally also possible to only connect one of the lumens, especially if a pump is chosen that provide a continuous flow of cooling solution.

In fact, it may be advantageous to use a third electrode connected directly to the fluid in the container 403 to ensure that no unwanted electrical potential differences will occur.

The container 403 is preferably made from a material with high dielectric breakdown, such as Teflon or Ultra High Molecular Weight Polyethylene. The container is also preferably closed and is naturally provided with means for filling and emptying (not shown) container. The pump 405 is preferably a magnetic impeller pump or a peristaltic pump, since both may easily be electrically isolated from the conducting solution. The catheter 407 is attached to the power unit such that the electrical conductor connected to the cathode of the x-ray tube (not shown) is connected via connection point 411 to the negative pole of the power source 402. The connection point 411 connects to the conducting layer 412 integrated into the catheter. This layer in turn is connected to the cathode of the x-ray tube.

Although no fluid is intended to enter the human body, it is practical to use a isoosmotic (0.9%) saline solution because it is very common in medical use. This solution has a resistivity of about 60ohmcm. The resulting series resistance in the solution is easily calculated to some 15MOhms for a 150cm catheter with two 0.1mm diameter lumen. This is not a problem as compared to the X-ray tube (operating at V=20,000V and I=5 microamperes typically). However, this resistance must be small compared to other resistances between the system and the human body for example. This resistance can be calculated for nylon, for instance, giving a resistance of the order of 10¹² Ohms between the lumen and the body. Therefore, also this requisite is met.

The connection to the miniature electrical x-ray tube is shown in more detail in figure 5. The distal end of the catheter 501 is provided with a small cavity 502 and the two lumens 503 and 504. The cavity is in direct physical and electrical contact with the miniature x-ray tube 505, and especially its anode 507. The x-ray tube comprises essentially a cathode 506 and an anode 507 in a vacuum chamber 508. The cathode is connected to a conductor 509, the conductor preferably essentially forming a coaxial arrangement with respect to the lumens 503 and 504.

According to a preferred embodiment applicable to both the first and second embodiments set forth above is the most distal end of the catheter provided with a hole 510 in which a guide wire 511 is adapted to pass. The device is threaded over the wire and is guided to its correct position.
The device may also be provided with radio opaque markers (not shown) in order identify and to be able to place the device at the correct position.

Of course, several modifications of the x-ray tube used in the x-ray tube unit according to the present invention are possible. For example, the x-ray tube could include grids, well known in themselves, to form a triode.

The present invention is not limited to the above-described preferred embodiments. Various alternatives, modifications and equivalents may be used. Therefore, the above embodiments should not be taken as limiting the scope of the invention, which is defined by the appending claims.

## Claims

1. An elongated x-ray tube unit comprising a miniaturized x-ray tube (2,505) arranged in a distal end of the unit adapted to generate x-ray radiation at a treatment position within a patient's body, **characterized in that** said x-ray tube unit further comprises a closed cooling solution supplying system provided with tubing means (24) arranged within the x-ray tube unit and adapted to provide a cooling solution to the vicinity of the x-ray tube in order to cool the x-ray tube.

2. X-ray tube unit according to claim 1,**characterized in that** the cooling solution supplying system is closed in the sense that no cooling solution is expelled from the system inside the patient's body.

3. X-ray tube unit according to claim 1 or2,**characterized in that** said cooling solution supplying system comprises a pumping arrangement on the proximal side of the x-ray tube unit and connected to the tubing means that runs along the x-ray tube unit to a cooling location close to the x-ray tube, wherein the pumping arrangement supplies the system with cooling solution.

4. X-ray tube unit according to any preceding claim, **characterized in that** the cooling solution is supplied, via said tubing means, to a cooling chamber (26) arranged close to the x-ray tube.

5. X-ray tube unit according to any preceding claim, **characterized in that** said tubing means comprises an inlet lumen (406,503) arranged to transport cooling solution to the x-ray tube and an outlet lumen (408,504) arranged to transport cooling solution away from the x-ray tube.

6. X-ray tube unit according to any preceding claim, **characterized in that** said cooling solution is saline.

7. X-ray tube unit according to any preceding claim, **characterized in that** said x-ray tube is electrically energized via said cooling solution.

8. X-ray tube unit according to claim 7,**characterized in that** said unit comprises a power source, arranged at the proximal end of the tube unit, with two poles, wherein one of said poles is electrically connected via the cooling solution to one of a first and a second electrodes of the x-ray tube and the other pole is electrically connected to the other electrode via an electrical conductor (509).

9. X-ray tube unit according to any preceding claim, **characterized in that** the cooling solution is provided to the one of a first and a second electrode of said x-ray tube that generates most heat.

10. X-ray tube unit according to any of claims 8 or 9, **characterized in that** the first electrode is the anode and the second electrode is the cathode.
